# EUROPEAN PATENT APPLICATION

(11) **EP 4 079 282 A1**
(43) Date of publication of application: **26.10.2022**
(21) Application number: 20901453.9
(22) Date of filing: 14.12.2020
(51) Int. Cl.: A61K 8/00, A61Q 11/00, A61Q 15/00, A61Q 19/10, G01N 33/497

(54) **METHOD FOR IMPROVING OLFACTORY SENSITIVITY**

(30) Priority: 19.12.2019 JP 2019229241
(71) Applicant: Kao Corporation, Chuo-ku Tokyo 103-8210 (JP)
(72) Inventor: TAKASE, Dan, Haga-gun, Tochigi 321-3497 (JP); YOSHIKAWA, Keiichi, Haga-gun, Tochigi 321-3497 (JP); OHASHI, Masatoshi, Tokyo 131-8501 (JP)
(74) Representative: Vossius & Partner Patentanwälte Rechtsanwälte mbB
(86) International application number: PCT/JP2020/046586
(87) International publication number: WO 2021/125139

(57) **Abstract**

The improvement of olfactory sensitivity. A method for improving olfactory sensitivity, comprising reducing body odor.

SEQUENCE LISTING

<110>  Kao Corporation

<120>  Method for improving olfactory sensitivity

<130>  AF2239 EP S3

<140>  EP 20 901 453.9
<141>  2020/12/14

<150>  JP 2019-229241
<151>  2019/12/19

<160>  5

<170>  PatentIn version 3.5


<210>  1
<211>  933
<212>  DNA
<213>  Homo sapiens

<220>
<223>  DNA encoding human OR10G4

<400>  1
atgtccaacg ccagcctcgt gacagcattc atcctcacag gccttcccca tgccccaggg      60

ctggacgccc tcctctttgg aatcttcctg gtggtttacg tgctcactgt gctggggaac     120

ctcctcatcc tgctggtgat cagggtggat tctcacctcc acacccccat gtactacttc     180

ctcaccaacc tgtccttcat tgacatgtgg ttctccactg tcacggtgcc caaaatgctg     240

atgaccttgg tgtccccaag cggcagggct atctccttcc acagctgcgt ggctcagctc     300

tattttttcc acttcctggg gagcaccgag tgtttcctct acacagtcat gtcctatgat     360

cgctacttgg ccatcagtta cccgctcagg tacaccagca tgatgagtgg gagcaggtgt     420

gccctcctgg ccaccggcac ttggctcagt ggctctctgc actctgctgt ccagaccata     480

ttgactttcc atttgcccta ctgtggaccc aaccagatcc agcactactt ctgtgacgca     540

ccgcccatcc tgaaactggc ctgtgcagac acctcagcca acgtgatggt catctttgtg     600

gacattggga tagtggcctc aggctgcttt gtcctgatag tgctgtccta tgtgtccatc     660

gtctgttcca tcctgcggat ccgcacctca gatgggaggc gcagagcctt tcagacctgt     720

gcctcccact gtattgtggt cctttgcttc tttgttccct gtgttgtcat ttatctgagg     780

ccaggctcca tggatgccat ggatggagtt gtggccattt tctacactgt gctgacgccc     840

cttctcaacc ctgttgtgta caccctgaga aacaaggagg tgaagaaagc tgtgttgaaa     900

cttagagaca aagtagcaca tcctcagagg aaa                                  933


<210>  2
<211>  933

<212>  DNA
<213>  Homo sapiens

<220>
<223>  DNA encoding human OR4S2

<400>  2
atggaaaaaa taaacaacgt aactgaattc attttctggg gtctttctca gagcccagag      60

attgagaaag tttgttttgt ggtgttttct ttcttctaca taatcattct tctgggaaat     120

ctcctcatca tgctgacagt ttgcctgagc aacctgttta agtcacccat gtatttcttt     180

ctcagcttct tgtcttttgt ggacatttgt tactcttcag tcacagctcc caagatgatt     240

gttgacctgt tagcaaagga caaaaccatc tcctatgtgg ggtgcatgtt gcaactgttt     300

ggagtacatt tctttggttg cactgagatc ttcatcctta ctgtaatggc ctatgatcgt     360

tatgtggcta tctgtaaacc cctacattat atgaccatca tgaaccggga gacatgcaat     420

aaaatgttat tagggacgtg ggtaggtggg ttcttacact ccattatcca agtggctctg     480

gtagtccaac tacccttttg tggacccaat gagatagatc actacttttg tgatgttcac     540

cctgtgttga aacttgcctg cacagaaaca tacattgttg gtgttgttgt gacagccaac     600

agtggtacca ttgctctggg gagttttgtt atcttgctaa tctcctacag catcatccta     660

gtttccctga gaaagcagtc agcagaaggc aggcgcaaag ccctctccac ctgtggctcc     720

cacattgcca tggtcgttat ctttttcggc ccctgtactt ttatgtacat gcgccctgat     780

acgacctttt cagaggataa gatggtggct gtattttaca ccattatcac tcccatgtta     840

aatcctctga tttatacact gagaaatgca gaagtaaaga atgcaatgaa gaaactgtgg     900

ggcagaaatg ttttcttgga ggctaaaggg aaa                                  933


<210>  3
<211>  933
<212>  DNA
<213>  Homo sapiens

<220>
<223>  DNA encoding human OR5P3

<400>  3
atggggactg gaaatgacac cactgtggta gagtttactc ttttggggtt atctgaggat      60

actacagttt gtgctatttt atttcttgtg tttctaggaa tttatgttgt caccttaatg     120

ggtaatatca gcataattgt attgatcaga agaagtcatc atcttcatac acccatgtac     180

attttcctct gccatttggc ctttgtagac attgggtact cctcatcagt cacacctgtc     240

atgctcatga gcttcctaag gaaagaaacc tctctccctg ttgctggttg tgtggcccag     300

ctctgttctg tagtgacgtt tggtacggcc gagtgcttcc tgctggctgc catggcctat     360

gatcgctatg tggccatctg ctcacccctg ctctactcta cctgcatgtc ccctggagtc     420

tgcatcatct tagtgggcat gtcctacctg ggtggatgtg tgaatgcttg gacattcatt 480

ggctgcttat taagactgtc cttctgtggg ccaaataaag tcaatcactt tttctgtgac 540

tattcaccac ttttgaagct tgcttgttcc catgatttta cttttgaaat aattccagct 600

atctcttctg gatctatcat tgtggccact gtgtgtgtca tagccatatc ctacatctat 660

atcctcatca ccatcctgaa gatgcactcc accaagggcc gccacaaggc cttctccacc 720

tgcacctccc acctcactgc agtcactctg ttctatggga ccattacctt catttatgtg 780

atgcccaagt ccagctactc aactgaccag aacaaggtgg tgtctgtgtt ctacaccgtg 840

gtgattccca tgttgaaccc cctgatctac agcctcagga acaaggagat taagggggct 900

ctgaagagag agcttagaat aaaaatattt tct 933

<210> 4
<211> 960
<212> DNA
<213> Homo sapiens

<220>
<223> DNA encoding human OR2W1

<400> 4
atggaccaaa gcaattatag ttctttacat ggttttattc tgcttggctt ctctaaccat 60

ccaaaaatgg agatgatcct gtcaggagtt gtcgccatct tctacttaat tacattggtg 120

ggtaacacag ccatcattct tgcatctctc ctggattccc agcttcatac accaatgtac 180

tttttcctca gaaatttatc tttcctagat ctatgtttca caaccagcat catccctcag 240

atgctggtca acttgtgggg acctgataag accatcagct atgtgggttg tatcatccaa 300

ctctatgttt acatgtggtt gggctcagtt gagtgccttc tcctggctgt tatgtcctat 360

gatcgtttta cagctatatg taagcccttg cattattttg tagtcatgaa cccacatcta 420

tgtctaaaga tgattatcat gatctggagt attagtttgg ccaattctgt agtattatgt 480

acactcactc tgaatttgcc cacatgtgga aacaacattc tggatcattt cttgtgtgag 540

ttgccagctc tggtcaagat agcttgtgta gacaccacaa cagttgaaat gtctgttttc 600

gctttaggca ttataattgt cctcacacct ctcatcctta ttcttatatc ctatggctac 660

attgccaaag ctgtgctgag aacgaagtca aaagcaagcc agcgaaaagc aatgaatacc 720

tgtggatctc atcttactgt agtgtctatg ttctatggaa ctattatcta catgtacctg 780

caaccaggta acagggcttc caaagaccag ggcaagttcc tcaccctctt ttacaccgtc 840

atcactccaa gtctcaaccc gctcatttac accttaagaa ataaggacat gaaggatgcc 900

ctgaagaaac tgatgagatt tcaccacaaa tctacaaaaa taaagaggaa ttgcaagtca 960

<210> 5
<211> 927

<212>  DNA
<213>  Homo sapiens

<220>
<223>  DNA encoding human OR1A1

<400>  5
atgagggaaa ataaccagtc ctctacactg gaattcatcc tcctgggagt tactggtcag      60

caggaacagg aagatttctt ctacatcctc ttcttgttca tttaccccat cacattgatt     120

ggaaacctgc tcatcgtcct agccatttgc tctgatgttc gccttcacaa ccccatgtat     180

tttctccttg ccaacctctc cttggttgac atcttcttct catcggtaac catccctaag     240

atgctggcca accatctctt gggcagcaaa tccatctctt ttgggggatg cctaacgcag     300

atgtatttca tgatagcctt gggtaacaca gacagctata ttttggctgc aatggcatat     360

gatcgagctg tggccatcag ccgcccactt cactacacaa caattatgag tccacggtct     420

tgtatctggc ttattgctgg gtcttgggtg attggaaatg ccaatgccct cccccacact     480

ctgctcacag ctagtctgtc cttctgtggc aaccaggaag tggccaactt ctactgtgac     540

attaccccct tgctgaagtt atcctgttct gacatccact ttcatgtgaa gatgatgtac     600

ctaggggttg gcattttctc tgtgccatta ctatgcatca ttgtctccta tattcgagtc     660

ttctccacag tcttccaggt tccttccacc aagggcgtgc tcaaggcctt ctccacctgt     720

ggttcccacc tcacggttgt ctctttgtat tatggtacag tcatgggcac gtatttccgc     780

cctttgacca attatagcct aaaagacgca gtgatcactg taatgtacac ggcagtgacc     840

ccaatgttaa atcctttcat ctacagtctg agaaatcggg acatgaaggc tgccctgcgg     900

aaactcttca acaagagaat ctcctcg                                         927

## Description

### Field of the Invention

The present invention relates to a method for improving olfactory sensitivity.

### Background of the Invention

Odor plays an important role for enjoying an affluent daily life. For example, odor provides rich taste to meals, and odor imparted to daily products provides various pleasantness such as cleanliness. On the other hand, unpleasant odor is also important. In order to promptly inform that health or sanitary conditions are damaged, such odor plays a great role. The importance of such odors can be assumed also from the facts that the tastes of meals are impaired when respiratory diseases such as cold are developed, and that elderly people with reduced sense of smell are exposed to risk because they cannot recognize the odor imparted to gas.

In mammals such as humans, odor is received by the olfactory receptors of olfactory sensory neurons that are present on the olfactory epithelium spreading in the deepest part of the nasal cavity. An odor substance incorporated into the nasal cavity acts on and activates olfactory receptors, and signals from the olfactory sensory neurons caused by the thus activated olfactory receptors are transmitted to the central nervous system, so that the odor can be perceived. It is presumed that 400 or more types of genes encoding such olfactory receptors are present in the case of humans. It is considered that the quality of odor of a specific odor substance which we perceive is determined depending on what combination of olfactory receptors among the above-described 400 or more olfactory receptors is activated by the odor substance. That is to say, individual olfactory receptors receive, with different affinity, a plurality of odor substances each having a similar structure, whereas individual odor substances are received by a plurality of olfactory receptors. Moreover, it has also been reported that an odor substance which activates a certain olfactory receptor inhibits the activation of another olfactory receptor. A combination of the responses of such a plurality of olfactory receptors brings on the recognition of each odor.

When a person continuously perceives a certain odor, his or her sensitivity to the certain odor is reduced. This phenomenon is referred to as "odor adaptation." Odor adaptation is classified into two types, namely, short-term adaptation and long-term adaptation, and it is considered that the molecular mechanisms causing these two adaptations are different from each other. In the short-term adaptation, olfactory sensitivity is reduced by exposure to an odor substance for several seconds to several minutes, and then, the sensitivity is promptly recovered if the odor substance disappears. It has been suggested that the cause of such a short-term adaptation should be a transient change in olfactory sensory neurons, such as the mechanism of losing responsiveness due to phosphorylation of an olfactory receptor which has responded to an odor (Non Patent Literature 1). If the odor substance disappears, the olfactory receptor is dephosphorylated, and the responsiveness thereof is thereby recovered. On the other hand, the long-term adaptation occurs in a unit of several weeks, and an equivalent period of time is also required for the recovery (Non Patent Literature 2). In Non Patent Literatures 3 and 4, it is suggested that a reduction in the expression level of the mRNA of an olfactory receptor that has been continuously exposed to an odor should cause such a long-term adaptation.

Furthermore, there may be a case where the sensitivity of a person to a certain odor is reduced when the person continuously perceives another odor. This phenomenon is referred to as "odor cross-adaptation". Patent Literature 1 discloses that activation of an olfactory receptor is associated with odor cross-adaptation, and that such cross-adaptation is observed among odor substances received by an identical olfactory receptor. That is, when the responsiveness of an olfactory receptor is reduced by being exposed to a certain odor substance, the responsiveness of the olfactory receptor to another odor substance is also reduced, and it is considered that this causes odor cross-adaptation. Non Patent Literature 5 discloses that perception to the odor of 2-furfurylthiol having a coffee-like odor is reduced or modulated by previously smelling the odor of furfuryl methyl sulfide that is a metabolite of 2-furfurylthiol, and that an olfactory receptor which responds to both of these compounds is associated with this cross-adaptation.

(Patent Literature 1) WO 2016/194788
(Non Patent Literature 1) Chem Senses, 2014, 39: 771-80
(Non Patent Literature 2) Percept Psychophys, 1996, 58: 781-792
(Non Patent Literature 3) Elife, 2017, e21476
(Non Patent Literature 4) Nat Commun, 2018, 9: 5081
(Non Patent Literature 5) Chem Senses, 2019, doi: 10.1093/chemse/bjz041

### Summary of the Invention

The present invention provides a method for immediately improving olfactory sensitivity of a subject, comprising reducing body odor of the subject.

In addition, the present invention provides a method for selecting an agent for immediately improving olfactory sensitivity, comprising specifying a substance which removes a body odor-causing substance.

Moreover, the present invention provides an agent for immediately improving olfactory sensitivity, comprising, as an active ingredient, a substance for removing an oral odor-causing substance from the oral cavity.

Furthermore, the present invention provides a method for evaluating body odor, comprising measuring olfactory sensitivity of a subject.

Further, the present invention provides a kit for evaluating body odor, comprising a means for measuring the olfactory sensitivity of a subject.

### Brief Description of the Drawings

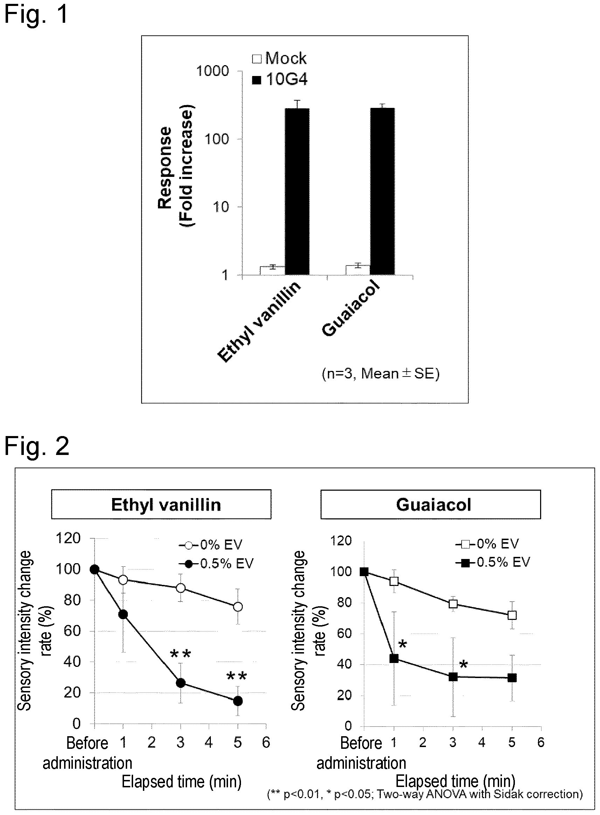

[Figure 1] Figure 1 shows the response of OR10G4 to Ethyl vanillin and Guaiacol. The horizontal axis indicates each test substance. The longitudinal axis indicates the response intensity of the olfactory receptor. Mock: Cells which do not express the olfactory receptor; and 10G4: OR10G4-expressing cells.
[Figure 2] Figure 2 shows a change over time in the odor sensory intensity of Ethyl vanillin (left view) and Guaiacol (right view), when subjects lick Ethyl vanillin-containing/-non-containing tablets. The horizontal axis indicates an elapsed time (min) after initiation of the licking of the tablet, and the longitudinal axis indicates a relative sensory intensity (%) when the odor sensory intensity of the odor substance immediately before the licking of the tablet is set as 100. 0.5% EV: 0.5% Ethyl vanillin-containing tablet; and 0% EV: Ethyl vanillin-non-containing tablet. In each case, n = 3, and error bar = ±SE. *: p < 0.05, **: p < 0.01 (a multiple comparison test according to the Sidak method, 0.5% EV vs 0% EV).

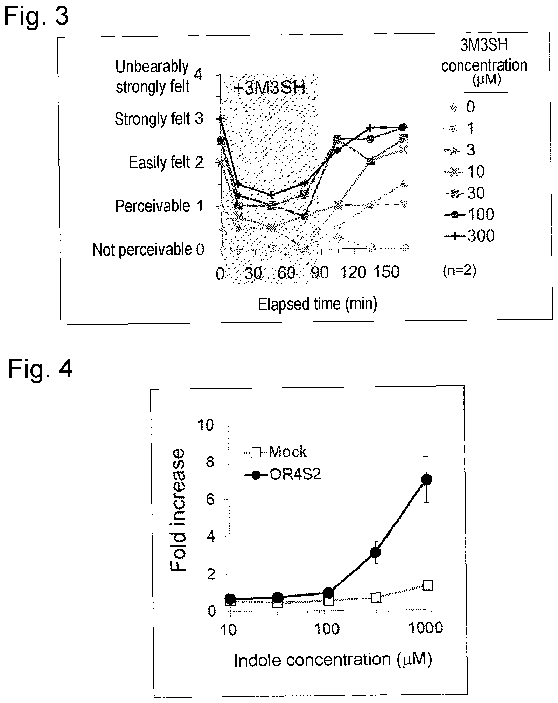

[Figure 3] Figure 3 shows a change over time in odor sensory intensity to 3M3SH, when 3M3SH is applied to the armpit portions of clothes. The horizontal axis indicates an elapsed time (min) after 3M3SH has been applied to both of the armpit portions of clothes for the first time; and the longitudinal axis indicates the odor sensory intensity score perceived to the 3M3SH sample (a mean value of two subjects). 0 (Zero) minute on the horizontal axis indicates the sensory intensity score immediately before application of 3M3SH. The shaded area in the figure indicates the time area in which 3M3SH is present in the armpit portions.
[Figure 4] Figure 4 shows the response of the olfactory receptor OR4S2 to various concentrations of indole. In each case, n = 3, and error bar = ±SE.

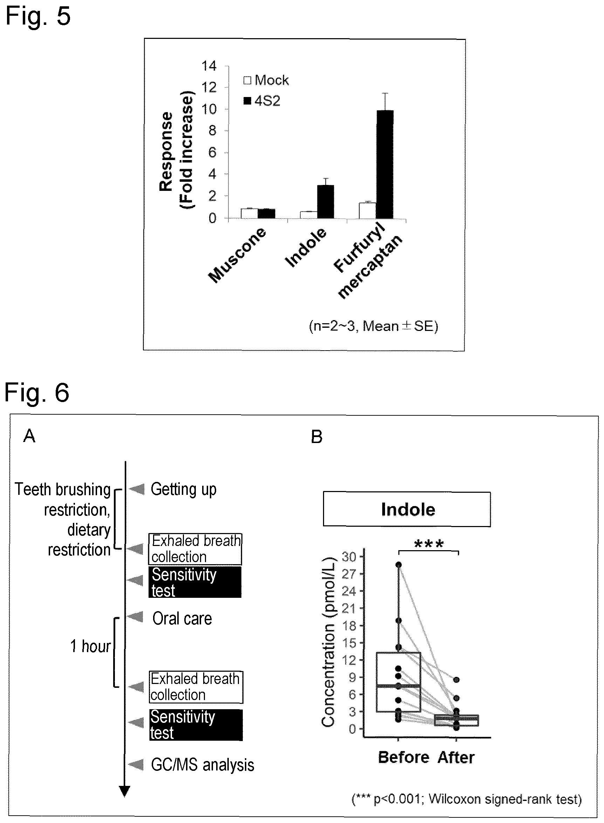

[Figure 5] Figure 5 shows the response of OR4S2 to muscone, indole, and furfuryl mercaptan. The horizontal axis indicates each test substance. The longitudinal axis indicates the response intensity of the olfactory receptor. Mock: Cells which do not express the olfactory receptor. 4S2: OR4S2-expressing cells.
[Figure 6] Figure 6A shows the experimental scheme of Example 3. Figure 6B shows a change in the indole concentrations in exhaled breathes before and after oral care. The longitudinal axis indicates the indole concentrations in the exhaled breathes (pmol/L). "Before" indicates before oral care (upon initiation of the test), and "After" indicates 1 hour after the oral care. The two plots connected with each other using a line indicate data from an identical subject. n = 14. ***: p < 0.001 (Wilcoxon signed-rank test).

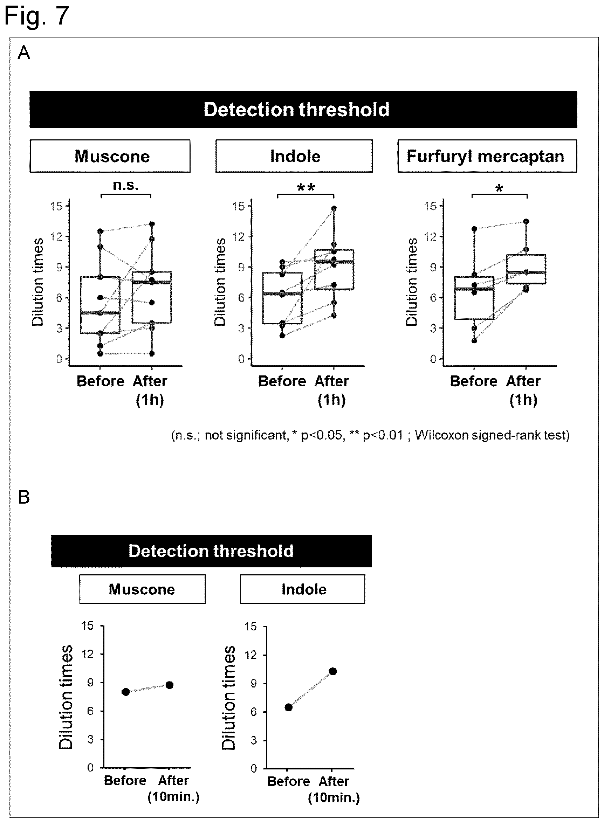

[Figure 7] Figure 7 shows a fluctuation in olfactory sensitivity to odor substances before and after oral care (Figure 7A: 1 hour after oral care; Figure 7B: 10 minutes after oral care). On the horizontal axis, "Before" indicates before oral care (upon initiation of the test), and "After" indicates after the oral care. The longitudinal axis indicates the detection threshold concentration of the odor substance (number of 2-fold dilutions from the maximum concentration). The two plots connected with each other using a line indicate data from an identical subject. In Figure 7A, n = 9 in the case of muscone, n = 8 in the case of indole, and n = 6 in the case of furfuryl mercaptan. *: p < 0.05, **: p < 0.01 (Wilcoxon signed-rank test).

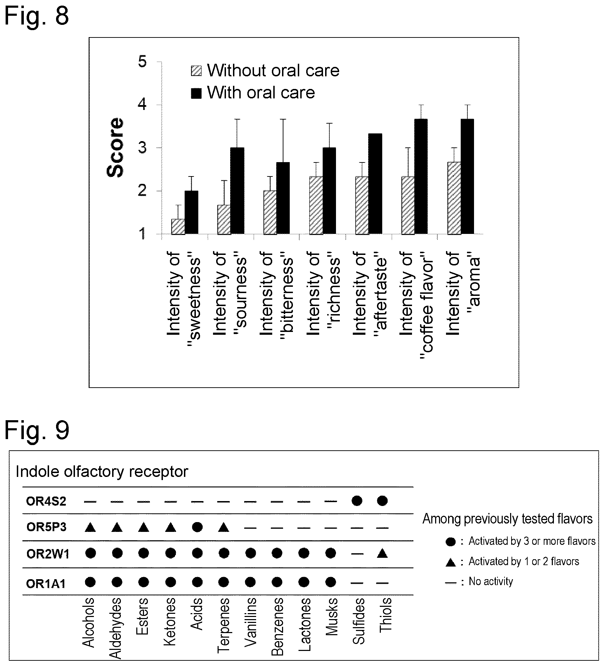

[Figure 8] Figure 8 shows the improvement of sensitivity to coffee flavor by oral care. The horizontal axis indicates evaluation items of sensory evaluation. The longitudinal axis indicates the sensory evaluation score (1: not felt, 2: not felt so much, 3: felt, 4: strongly felt, 5: extremely strongly felt). n = 3, and error bar = SE.
[Figure 9] Figure 9 shows ligand spectra of indole receptors. Specifically, this figure shows classification of the chemical structures of odor substances, to which the indole receptors OR4S2, OR5P3, OR2W1 and OR1A1 respond. The filled circle: the receptor activated by 3 or more odor substances included in this classification; the filled triangle: the receptor activated by 1 or 2 odor substances included in this classification; and -: no odor substances which activates receptors are found in this classification.

### Detailed Description of the Invention

Herein, the term "odor cross-adaptation" regarding a target odor means a phenomenon in which the olfactory sensitivity of a subject to the target odor is reduced or changed when the subject has previously received an odor of a substance that is different from a causing substance of the target odor, and has habituated to the odor. In Patent Literature 1, it is elucidated that the "odor cross-adaptation" is a phenomenon based on olfactory receptor agonism. Specifically, an olfactory receptor to a causing substance of a target odor has responded to a causing substance of a different odor, before responding to the causing substance of the target odor, and thereafter, the responsiveness thereof is reduced, and thus, the olfactory receptor can respond only at a low level even if it is exposed to the causing substance of the target odor later, thereby resulting in a reduced or altered intensity of the target odor which is recognized by an individual.

The term "improvement of olfactory sensitivity" as used herein means that detectable intensity to the target odor is increased, or that a detection threshold to the target odor is reduced.

The names of the olfactory receptors as used herein are names registered in Genbank (/www.ncbi.nlm.nih.gov/genbank/).

The term "body odor" as used herein means an odor constantly present in an individual, which is generated from the body of the individuals or clothes to wear, etc. Examples of the body odor may include, but are not particularly limited to, scalp odor, oral odor, foot odor, sebum odor, underarm odor, sweat odor, and aging odor.

The odor to which a human is continuously exposed most frequently may be the body odor of the human. The body odor of a human continuously acts on the human sense of smell, and it should cause odor adaptation. Indeed, it is an empirically well-known fact that a human hardly perceives his or her own body odor. In addition, although it has not been demonstrated so far, when adaptation to a body odor has occurred, the aforementioned cross-adaptation has also occurred simultaneously, and it is assumed that not only sensitivity to the body odor, but also sense of smell to a substance group other than the body odor is rendered poor.

Conventionally, it has been assumed that since olfactory sensory neurons are constantly exposed to the body odor, the above-described odor adaptation and cross-adaptation caused by body odor would be due to long-term adaptation. Thus, it has been predicted that a long period of time would be required for recovery from these adaptation and cross-adaptation. In contrast to this prediction, the present inventors found that an individual can recover from the odor adaptation and cross-adaptation caused by the body odor of the individual in a relatively short time by reducing the body odor of the individual, and that the olfactory sensitivity of the individual can be improved.

Furthermore, it was also found that the above-described cross-adaptation with body odor is generated with respect to an odor substance which activates the same olfactory receptor as the olfactory receptor that responds to the body odor. Therefore, according to the present invention, by reducing the body odor of an individual, the olfactory sensitivity of the individual to another odor substance which activates the same olfactory receptor as the olfactory receptor that responds to the body odor can be improved in a relatively short time.

The above-described results show that temporary body odor care improves the olfactory sensitivity of an individual. Such a technique of improving olfactory sensitivity would be preferable to those in need of the improvement of olfactory sensitivity, preferably, those in need of the improvement of olfactory sensitivity by a reduction in the body odor, for example, the middle-aged and elderly people who would generally increase body odor and would tend to reduce sense of smell (but are not limited thereto).

In addition, the above-described results show that a substance or means for contributing to body odor care even temporally (e.g. washing or a deodorant for body odor) is effective for the improvement of olfactory sensitivity. By applying the substance or means for contributing to body odor care to an individual, the olfactory sensitivity of the individual can be improved.

Moreover, the above-described results show that the intensity of body odor is reflected on the olfactory sensitivity of an individual. In other words, it is possible to evaluate the body odor of an individual, using its olfactory sensitivity as an indicator. Specifically, if the olfactory sensitivity of an individual is used as an indicator, it is possible to evaluate, by him or herself, the intensity of body odor which is hardly perceived by his or her own sense of smell due to adaptation.

The present invention relates to a method for improving the olfactory sensitivity of an individual. The present inventors found that, via an olfactory receptor which responds to body odor, the body odor of an individual affects the olfactory sensitivity of the individual to another substance which activates the same olfactory receptor.

The present invention provides a method for improving the olfactory sensitivity of an individual by controlling the body odor of the individual. In addition, the present invention provides various means or substances capable of controlling the body odor of an individual, and further, capable of improving the olfactory sensitivity of the individual. Moreover, the present invention provides a method for evaluating the body odor of an individual, using the olfactory sensitivity of the individual as an indicator, and a kit therefor.

Accordingly, in one aspect, the present invention provides a method for improving the olfactory sensitivity of a subject, comprising reducing the body odor of the subject.

The means for reducing the body odor of a subject may be, for example, removal of a body odor-causing substance from the body of the subject, clothes to wear, etc. The means for removing a body odor-causing substance may include washing, adsorption, generation inhibition, volatilization inhibition, decomposition, etc. of a body odor-causing substance. The washing of a body odor-causing substance may include washing-out of a body odor-causing substance from the body or clothes, using water or a detergent for bodies or clothes. The adsorption, generation inhibition, volatilization inhibition, or decomposition of a body odor-causing substance may include application of activated carbon, a disinfectant, an antibacterial agent, a metabolic inhibitor or an enzyme inhibitor against body odor generation-causing bacteria, an antioxidant, cyclic dextrin, a chemical neutralizer, a deodorant, etc. to the body or clothes.

Examples of the body odor-causing substance regarding scalp odor may include diacetyl and isovaleric acid; examples of the body odor-causing substance regarding oral odor may include the substances listed in Table 3 below, such as indole and skatole; an example of the body odor-causing substance regarding foot odor may be isovaleric acid; examples of the body odor-causing substance regarding sebum odor may include unsaturated aliphatic aldehydes having 7 to 10 carbon atoms, such as 2,4-heptadienal, 2-octenal, 2-nonenal, 2-decenal, and 2,4-decadienal; examples of the body odor-causing substance regarding underarm odor may include 3-methyl-2-hexenoic acid, 3-hydroxy-3-methylhexanoic acid, and 3-methyl-3-sulfanylhexan-1-ol (3M3SH); examples of the body odor-causing substance regarding sweat odor may include 4-methylpentanoic acid, 4-methyl-2-pentenoic acid, 4-methyl-3-pentenoic acid, and 4-methyl-4-pentenoic acid; and examples of the body odor-causing substance regarding aging odor may include 2-nonenal, n-octanal, n-nonanal, and n-decanal.

As a result of the body odor reduction, the olfactory sensitivity of a subject to an odor substance having adaptation or cross-adaptation to the body odor is improved. More specifically, in the present invention, as a result of the body odor reduction, the olfactory sensitivity of a subject to an odor substance which activates an olfactory receptor that responds to a causing substance of the body odor is improved. Preferably, the odor substance to which the olfactory sensitivity of a subject is improved in the present invention is an odor substance other than the body odor-causing substance. In the present invention, the improvement of the olfactory sensitivity of a subject to the odor substance is achieved in a relatively short time after the treatment of reducing the body odor has been carried out. Therefore, the improvement of olfactory sensitivity according to the present invention may be immediate improvement. The "immediate" improvement of olfactory sensitivity according to the present invention means that the improvement of olfactory sensitivity occurs preferably within 1 hour, more preferably within 10 minutes, after the treatment of reducing the body odor.

In a preferred embodiment, the body odor is oral odor. Examples of a means for reducing such oral odor may include:, by use of water, or an oral care product such as a dentifrice, mouth wash, an oral tablet, or an oral spray, washing-out of an oral odor-causing substance from the oral cavity, adsorption or decomposition of an oral odor-causing substance, sterilization of oral odor causing-bacteria, metabolic inhibition or enzyme inhibition against oral odor causing-bacteria, suppression of formation of or decomposition of a biofilm containing oral odor causing-bacteria; and washing or sterilization of dentures. As described in the after-mentioned Examples (see Table 3), various substances are present as oral odor-causing substances, and also, olfactory receptors which respond to individual oral odor-causing substances are present. Examples of the olfactory receptors which respond to the oral odor-causing substances may include OR4S2, OR5P3, OR2W1, OR1A1, OR2T11, OR8H1, OR5K1, OR51E1, OR51E2, OR51I2, OR52N2, OR11G2, OR5AC2, OR11H6, OR11H4, OR4C15, OR8S1, OR1E1, OR8U8, OR51D1, OR51D14, OR2T1, OR5AI1, OR7G2, OR11H7P, and TAAR5. In the present invention, examples of the odor substance to which the olfactory sensitivity of a subject is improved as a result of the oral odor reduction may include odor substances which activate the aforementioned olfactory receptors which respond to oral odor-causing substances. Preferably, the odor substances to which the olfactory sensitivity is improved are odor substances other than the oral odor-causing substances.

In a preferred embodiment, the oral odor-causing substance is indole, and the odor substances to which the olfactory sensitivity of a subject is improved may include odor substances other than indole which activate indole receptors. Examples of the indole receptors may include OR4S2, OR5P3, OR2W1, and OR1A1. As described in the Examples below, immediately after, preferably within 1 hour after, and more preferably within 10 minutes to 1 hour after the oral odor of an individual has been reduced, the olfactory sensitivity of the individual to furfuryl mercaptan which activates the indole receptor OR4S2, and a coffee flavor containing, as a main ingredient, furfuryl mercaptan, is improved.

In another preferred embodiment, the body odor is underarm odor. A means for reducing such underarm odor may be, for example, removal of an underarm odor-causing substance from the body or clothes by washing the body (armpit portion) or clothes (in particular, armpit portion). In the present invention, examples of the odor substance to which the olfactory sensitivity of a subject is improved as a result of the underarm odor reduction may include odor substances other than underarm odor-causing substances which activate underarm odor receptors, such as, OR1A1, OR2W1, OR2J3, OR10A6, OR51B2, OR51E1, or OR51I2.

In another aspect, the present invention provides a means for improving the olfactory sensitivity of a subject. Preferably, the means for improving the olfactory sensitivity of a subject may be a means for removing a body odor-causing substance, for example, a means for washing, adsorption, generation inhibition, volatilization inhibition, or decomposition a body odor-causing substance. More specific examples of the means for removing a body odor-causing substance may include washing-out of a body odor-causing substance from a body or clothes, using water or a detergent for bodies or clothes, and application of activated carbon, a disinfectant, an antibacterial agent, a metabolic inhibitor or an enzyme inhibitor against body odor generation-causing bacteria, an antioxidant, cyclic dextrin, a chemical neutralizer, a deodorant, etc. to bodies or clothes for adsorption, generation inhibition, volatilization inhibition and decomposition of a body odor-causing substance. By these means for removing a body odor-causing substance, the body odor of a subject is removed, so that the olfactory sensitivity of the subject is immediately improved.

In one embodiment, the present invention provides an agent for improving olfactory sensitivity, containing, as an active ingredient, a substance for removing an oral odor-causing substance from the oral cavity. Preferably, the improving agent is an agent for improving olfactory sensitivity to an odor substance which activates an olfactory receptor that responds to the oral odor-causing substance. Preferably, the agent for improving olfactory sensitivity may be an agent for immediately improving olfactory sensitivity. Preferably, the active ingredient is a substance which washes, adsorbs, inhibits generation of, inhibits volatilization of, or decomposes an oral odor-causing substance. Preferably, the oral odor-causing substance is indole. Examples of the odor substance which activates an olfactory receptor that responds to the oral odor-causing substance may include odor substances which activate the indole receptor OR4S2, OR5P3, OR2W1 or OR1A1, and more preferred examples thereof may include furfuryl mercaptan, and a coffee flavor containing, as a main ingredient, furfuryl mercaptan.

In another embodiment, the present invention provides an agent for improving olfactory sensitivity, containing, as an active ingredient, a substance for removing an underarm odor-causing substance from the body (mainly, an armpit portion). Preferably, the improving agent is an agent for improving olfactory sensitivity to an odor substance which activates an olfactory receptor that responds to the underarm odor-causing substance. Preferably, the agent for improving olfactory sensitivity may be an agent for immediately improving olfactory sensitivity. Preferably, the active ingredient is a substance which washes, adsorbs, inhibits generation of, inhibits volatilization of, or decomposes an underarm odor-causing substance. Preferably, the underarm odor-causing substance is 3-methyl-2-hexenoic acid, 3-hydroxy-3-methylhexanoic acid, or 3-methyl-3-sulfanylhexan-1-ol (3M3SH). Examples of the odor substance which activates an olfactory receptor that responds to the underarm odor-causing substance may include odor substances which activate underarm odor receptors (e.g. OR1A1, OR2W1, OR2J3, OR10A6, OR51B2, OR51E1, or OR51I2).

Examples of the substance for removing an oral odor-causing substance from the oral cavity may include oral care products such as a dentifrice, mouth wash, an oral tablet, or an oral spray, and denture cleansers. The oral care products may optionally contain the following components, but the components are not limited to thereto: base materials or moisturizers, such as water, sorbitol, propylene glycol, glycerin, and ethanol; fluorines (sodium fluoride, sodium monofluorophosphate, etc.); disinfectants, such as benzethonium chloride, benzalkonium chloride, cetylpyridinium chloride, isopropylmethylphenol, chlorhexidine hydrochloride, and triclosan; copper, zinc or aluminum salts, such as copper gluconate, copper citrate, copper sulfate, copper chloride, sodium copper chlorophyllin, zinc gluconate, zinc citrate, zinc sulfate, zinc chloride, zinc oxide, aluminum hydroxide, and aluminum lactate; thickeners, such as silicic acid anhydride (thickening silica, etc.), carboxymethyl cellulose, carrageenan, xanthan gum, and polyacrylic acid; abrasive agents, such as silicic acid anhydride (abrasive silica, etc.) and calcium carbonate; activators, such as sodium lauryl sulfate, sodium lauroyl methyl taurine, sodium lauroyl glutamate, sodium myristoyl glutamate, coconut oil fatty acid amide propylbetaine, polyoxyethylene hydrogenated castor oil, polyoxyethylene sorbitan stearate, glycerin fatty acid ester, sucrose fatty acid ester, and polyoxyethylene stearyl ether; aromas or sweeteners, such as menthol and sodium saccharin; antiseptics such as paraben; cyclodextrin; and zeolite.

Furthermore, oral odor-preventing components which can be used in oral care products can themselves be used in the present invention, as a means for removing a body odor-causing substance used in the method for improving olfactory sensitivity, or as an active ingredient of an agent for improving olfactory sensitivity. Example of the oral odor-preventing components usable in oral care products may include: disinfectants, such as benzethonium chloride, benzalkonium chloride, cetylpyridinium chloride, isopropylmethylphenol, chlorhexidine hydrochloride, and triclosan; copper, zinc or aluminum salts, such as copper gluconate, copper citrate, copper sulfate, copper chloride, sodium copper chlorophyllin, zinc gluconate, zinc citrate, zinc sulfate, zinc chloride, zinc oxide, aluminum hydroxide, and aluminum lactate; activators, such as sodium lauryl sulfate, sodium lauroyl methyl taurine, sodium lauroyl glutamate, sodium myristoyl glutamate, coconut oil fatty acid amide propylbetaine, polyoxyethylene hydrogenated castor oil, polyoxyethylene sorbitan stearate, glycerin fatty acid ester, sucrose fatty acid ester, and polyoxyethylene stearyl ether; cyclodextrin; and zeolite. These oral odor-preventing components can be used, alone or in appropriate combination, as an active ingredient of the agent for improving olfactory sensitivity of the present invention.

In a further aspect, the present invention provides a method for selecting an agent for improving olfactory sensitivity. As described above, by removing a body odor-causing substance from a subject, the olfactory sensitivity of the subject can be immediately improved. Thus, a substance which removes a body odor-causing substance may be specified as an agent for improving olfactory sensitivity. Preferably, the substance which removes a body odor-causing substance is an agent for improving olfactory sensitivity to an odor substance which activates an olfactory receptor that responds to the body odor-causing substance. Preferably, the agent for improving olfactory sensitivity may be an agent for immediately improving olfactory sensitivity.

In one embodiment, the method for selecting an agent for improving olfactory sensitivity of the present invention includes examining the action of a test substance to remove a body odor-causing substance as a target. For example, in this method, the action of the test substance to wash, adsorb, inhibit generation of, inhibit volatilization of, or decompose the body odor-causing substance is examined. The test substance having the aforementioned action can be specified as an agent for improving olfactory sensitivity. A substance having a stronger effect of removing a body odor-causing substance can function as a stronger agent for improving olfactory sensitivity. The type of the test substance is not particularly limited, as long as it is a substance desired to be used as an agent for improving olfactory sensitivity. For example, the test substance may be a substance which is expected to have an action to wash, adsorb, inhibit generation of, inhibit volatilization of, or decompose a body odor-causing substance. The test substance may be either a naturally occurring substance, or a substance artificially synthesized by a chemical or biological method, etc. Also, the test substance may be a compound, a composition, or a mixture. In addition, the test substance may also be a substance whose action to improve olfactory sensitivity, or whose action to wash, adsorb, inhibit generation of, inhibit volatilization of, or decompose a body odor-causing substance has been evaluated or specified so far.

In another embodiment, the method for selecting an agent for improving olfactory sensitivity of the present invention includes performing sensory evaluation of the olfactory sensitivity-improving action of a substance which is specified to have an action to remove a body odor-causing substance. For example, as described above, a substance which removes a body odor-causing substance may be specified among test substances, and may be then subjected to sensory evaluation in terms of the action of the substance to improve olfactory sensitivity. Otherwise, a substance which has already been specified as a substance which removes a body odor-causing substance, may be subjected to sensory evaluation in terms of its action to improve olfactory sensitivity.

In a further aspect, the present invention provides a method for evaluating body odor, including measuring the olfactory sensitivity of a subject. In a further aspect, the present invention provides a kit for evaluating body odor, containing a means for measuring the olfactory sensitivity of a subject. As described in Examples below, the body odor of an individual affects his or her olfactory sensitivity. Accordingly, it is possible to evaluate the body odor of an individual, using the olfactory sensitivity as an indicator.

In the evaluation of body odor according to the present invention, the olfactory sensitivity of a subject to an odor substance which activates an olfactory receptor that responds to a body odor-causing substance used as an evaluation target (hereinafter referred to as a "test odor substance") is measured. The test odor substance is preferably an odor substance other than the body odor-causing substance used as an evaluation target. For example, when oral odor is to be evaluated, furfuryl mercaptan which activates a receptor of a representative oral odor-causing substance indole or a coffee flavor containing, as a main ingredient, furfuryl mercaptan can be used as a test odor substance. Moreover, for example, when underarm odor is to be evaluated, an odor substance which activates an underarm odor receptor (e.g. OR1A1, OR2W1, OR2J3, OR10A6, OR51B2, OR51E1, or OR51I2) can be used as a test odor substance.

In one embodiment of the evaluation of body odor according to the present invention, a subject is allowed to smell the odor of a sample containing a test odor substance in a predetermined concentration, and whether or not the subject perceives the odor is determined. The concentration of the test odor substance in the sample can be adjusted, as appropriate, depending on the age, sex, etc. of the subject. When the subject cannot perceive the odor of the test odor substance from the sample, the subject can be evaluated to have body odor (positive). On the other hand, when the subject can perceive the odor of the test odor substance from the sample, the subject can be evaluated to have no problems regarding body odor (negative).

In another embodiment of the evaluation of body odor according to the present invention, a subject is allowed to smell the odors of samples each containing a test odor substance in a stepwise different concentration, and the subject is then allowed to select a sample from which the subject perceives the odor. The concentration of the test odor substance in each sample can be adjusted, as appropriate, depending on the age, sex, etc. of the subject. Using, as a reference, the concentration of the test odor substance in the sample selected by the subject, the degree of body odor of the subject can be evaluated.

The kit for evaluating body odor according to the present invention is a kit for carrying out the aforementioned method for evaluating body odor according to the present invention. The kit contains a sample containing the test odor substance. Preferably, the sample is preferably hermetically sealed or coated until use, so that volatilization of the contained test odor substance can be prevented. For example, the sample is a card containing the test odor substance, and the surface of the card is coated in order to prevent volatilization of the contained test odor substance. Upon the evaluation of body odor, the coating is peeled, and the sample is then used. Preferably, the kit is a kit for evaluating oral odor, and the kit contains a sample containing, as a test odor substance, furfuryl mercaptan, or a coffee flavor containing, as a main ingredient, the furfuryl mercaptan. As another example, the kit is a kit for evaluating underarm odor, and the kit contains a sample containing, as a test odor substance, an odor substance which activates an underarm odor receptor (e.g. OR1A1, OR2W1, OR2J3, OR10A6, OR51B2, OR51E1, or OR51I2).

The kit for evaluating body odor may further contain a guidance for evaluating the body odor of a subject based on the olfactory sensitivity of the subject to a test odor substance. In one example, the guidance instructs, such that when a subject could perceive the odor of a test odor substance, the subject can be evaluated to have body odor, on the other hand, when the subject can perceive the odor of the test odor substance from the sample, the subject can be evaluated to have no problems regarding body odor. In another example, the guidance provides a reference for evaluating the degree of body odor of a subject, based on the concentration of a test odor substance which the subject could perceive. The guidance can be contained in a recording medium such as a paper, a magnetic disk, or a USB memory.

According to the method for evaluating body odor and the kit for evaluating body odor of the present invention, the intensity of the body odor of a subject which is hardly perceived by himself or herself due to adaptation can be evaluated by the subject himself or herself.

As illustrative embodiments of the present invention, the following compositions, production methods, intended uses, or methods will be further disclosed in the present description. However, the present invention is not limited to these embodiments.
[1] A method for immediately improving the olfactory sensitivity of a subject, comprising reducing body odor of the subject.
[2] The method according to the above [1], preferably comprising removing a body odor-causing substance from the body or clothes of the subject.
[3] The method according to the above [1] or [2], wherein
   the body odor is preferably scalp odor, oral odor, foot odor, sebum odor, underarm odor, sweat odor, or aging odor,
   more preferably oral odor or underarm odor, and
   further preferably oral odor.
[4] The method according to the above [3], wherein
   the reducing body odor preferably comprises washing-out of the body odor-causing substance from the body or clothes of the subject, and adsorption, generation inhibition, volatilization inhibition or decomposition of the body odor-causing substance in the body or clothes of the subject,
   more preferably comprises application of activated carbon, a disinfectant, an antibacterial agent, a metabolic inhibitor or an enzyme inhibitor against body odor generation-causing bacteria, an antioxidant, cyclic dextrin, a chemical neutralizer, or a deodorant to the bodies or clothes of the subject,
   further preferably comprises washing of the armpit portion of the body or clothes (preferably, the armpit portion), or comprises washing of the oral cavity, adsorption or decomposition of an oral odor-causing substance, sterilization of oral odor causing-bacteria, metabolism inhibition or enzyme inhibition against oral odor causing-bacteria, suppression of formation or decomposition of a biofilm comprising oral odor causing-bacteria, and washing or sterilization of dentures, and
   still further preferably comprises application of an oral odor-preventing component usable in oral care products into the oral cavity, wherein
   the oral odor-preventing component comprises at least one selected from the group consisting of a disinfectant, a copper, zinc or aluminum salt, an activator, cyclodextrin, and zeolite, which are usable in oral care products,
   the disinfectant comprises at least one selected from the group consisting of benzethonium chloride, benzalkonium chloride, cetylpyridinium chloride, isopropylmethylphenol, chlorhexidine hydrochloride, and triclosan,
   the copper, zinc or aluminum salt comprises at least one selected from the group consisting of copper gluconate, copper citrate, copper sulfate, copper chloride, sodium copper chlorophyllin, zinc gluconate, zinc citrate, zinc sulfate, zinc chloride, zinc oxide, aluminum hydroxide, and aluminum lactate, and
   the activator comprises at least one selected from the group consisting of sodium lauryl sulfate, sodium lauroyl methyl taurine, sodium lauroyl glutamate, sodium myristoyl glutamate, coconut oil fatty acid amide propylbetaine, polyoxyethylene hydrogenated castor oil, polyoxyethylene sorbitan stearate, glycerin fatty acid ester, sucrose fatty acid ester, and polyoxyethylene stearyl ether.
[5] The method according to any one of the above [1] to [4], wherein the olfactory sensitivity is preferably sensitivity to furfuryl mercaptan or coffee flavor.
[6] A method for selecting an agent for immediately improving olfactory sensitivity, comprising specifying a substance which removes a body odor-causing substance.
[7] A method for selecting an agent for immediately improving olfactory sensitivity, comprising performing sensory evaluation of the olfactory sensitivity-improving action of a substance specified as a substance which removes a body odor-causing substance.
[8] The method according to the above [6] or [7], wherein the substance which removes a body odor-causing substance is preferably a substance having an action to wash, adsorb, inhibit generation of, inhibit volatilization of, or decompose the body odor-causing substance.
[9] The method according to any one of the above [6] to [8], wherein
   the body odor is preferably scalp odor, oral odor, foot odor, sebum odor, underarm odor, sweat odor, or aging odor,
   more preferably oral odor or underarm odor, and
   further preferably oral odor.
[10] An agent for immediately improving olfactory sensitivity, comprising, as an active ingredient, a substance for removing an oral odor-causing substance from the oral cavity.
[11] The agent according to the above [10], wherein the substance for removing an oral odor-causing substance is preferably a substance which washes, adsorbs, inhibits generation of, inhibits volatilization of, or decompose the oral odor-causing substance.
[12] The agent according to the above [10] or [11], wherein the oral odor-causing substance is preferably indole.
[13] The agent according to any one of the above [10] to [12], wherein
   the substance for removing an oral odor-causing substance is preferably an oral odor-preventing component usable in oral care products,
   the oral odor-preventing component comprises at least one selected from the group consisting of a disinfectant, a copper, zinc or aluminum salt, an activator, cyclodextrin, and zeolite, which are usable in oral care products,
   the disinfectant comprises at least one selected from the group consisting of benzethonium chloride, benzalkonium chloride, cetylpyridinium chloride, isopropylmethylphenol, chlorhexidine hydrochloride, and triclosan,
   the copper, zinc or aluminum salt comprises at least one selected from the group consisting of copper gluconate, copper citrate, copper sulfate, copper chloride, sodium copper chlorophyllin, zinc gluconate, zinc citrate, zinc sulfate, zinc chloride, zinc oxide, aluminum hydroxide, and aluminum lactate, and
   the activator comprises at least one selected from the group consisting of sodium lauryl sulfate, sodium lauroyl methyl taurine, sodium lauroyl glutamate, sodium myristoyl glutamate, coconut oil fatty acid amide propylbetaine, polyoxyethylene hydrogenated castor oil, polyoxyethylene sorbitan stearate, glycerin fatty acid ester, sucrose fatty acid ester, and polyoxyethylene stearyl ether.
[14] Use of a substance for removing an oral odor-causing substance from the oral cavity for the immediate improvement of olfactory sensitivity.
[15] Use of a substance for removing an oral odor-causing substance from the oral cavity for the production of an agent for immediately improving olfactory sensitivity.
[16] The use according to the above [14] or [15], wherein the substance for removing an oral odor-causing substance is preferably a substance which washes, adsorbs, inhibits generation of, inhibits volatilization of, or decomposes the oral odor-causing substance.
[17] The use according to any one of the above [14] to [16], wherein the oral odor-causing substance is preferably indole.
[18] The use according to any one of the above [14] to [17], wherein
   the substance for removing an oral odor-causing substance is preferably an oral odor-preventing component usable in oral care products,
   the oral odor-preventing component comprises at least one selected from the group consisting of a disinfectant, a copper, zinc or aluminum salt, an activator, cyclodextrin, and zeolite, which are usable in oral care products,
   the disinfectant comprises at least one selected from the group consisting of benzethonium chloride, benzalkonium chloride, cetylpyridinium chloride, isopropylmethylphenol, chlorhexidine hydrochloride, and triclosan,
   the copper, zinc or aluminum salt comprises at least one selected from the group consisting of copper gluconate, copper citrate, copper sulfate, copper chloride, sodium copper chlorophyllin, zinc gluconate, zinc citrate, zinc sulfate, zinc chloride, zinc oxide, aluminum hydroxide, and aluminum lactate, and
   the activator comprises at least one selected from the group consisting of sodium lauryl sulfate, sodium lauroyl methyl taurine, sodium lauroyl glutamate, sodium myristoyl glutamate, coconut oil fatty acid amide propylbetaine, polyoxyethylene hydrogenated castor oil, polyoxyethylene sorbitan stearate, glycerin fatty acid ester, sucrose fatty acid ester, and polyoxyethylene stearyl ether.
[19] An agent for immediately improving olfactory sensitivity, comprising, as an active ingredient, a substance for removing an underarm odor-causing substance from the body.
[20] The agent according to the above [19], wherein the substance for removing an underarm odor-causing substance is preferably a substance which washes, adsorbs, inhibits generation of, inhibits volatilization of, or decomposes the underarm odor-causing substance.
[21] The agent according to the above [19] or [20], wherein the underarm odor-causing substance is preferably 3-methyl-2-hexenoic acid, 3-hydroxy-3-methylhexanoic acid, or 3-methyl-3-sulfanylhexan-1-ol.
[22] Use of a substance for removing an underarm odor-causing substance from the body for the immediate improvement of olfactory sensitivity.
[23] Use of a substance for removing an underarm odor-causing substance from the body for the production of an agent for immediately improving olfactory sensitivity.
[24] The use according to the above [22] or [23], wherein the substance for removing an underarm odor-causing substance is preferably a substance which washes, adsorbs, inhibits generation of, inhibits volatilization of, or decomposes the underarm odor-causing substance.
[25] The use according to any one of the above [22] to [24], wherein the underarm odor-causing substance is preferably 3-methyl-2-hexenoic acid, 3-hydroxy-3-methylhexanoic acid, or 3-methyl-3-sulfanylhexan-1-ol.
[26] A method for evaluating body odor, comprising measuring olfactory sensitivity of a subject.
[27] The method according to the above [26], wherein
   the body odor is preferably scalp odor, oral odor, foot odor, sebum odor, underarm odor, sweat odor, or aging odor,
   more preferably oral odor or underarm odor, and
   further preferably oral odor.
[28] The method according to the above [26] or [27], wherein the olfactory sensitivity is preferably sensitivity to furfuryl mercaptan or coffee flavor.
[29] A kit for evaluating body odor, comprising a means for measuring olfactory sensitivity of a subject.
[30] The kit according to the above [29], wherein
   the body odor is preferably scalp odor, oral odor, foot odor, sebum odor, underarm odor, sweat odor, or aging odor,
   more preferably oral odor or underarm odor, and
   further preferably oral odor.
[31] The kit according to the above [29] or [30], wherein the olfactory sensitivity is preferably sensitivity to furfuryl mercaptan or coffee flavor.
[32] The kit according to any one of the above [29] to [31], preferably comprising a sample comprising an odor substance which activates an olfactory receptor that responds to a body odor-causing substance used as an evaluation target, wherein the odor substance is preferably furfuryl mercaptan or coffee flavor.
[33] The kit according to the above [32], preferably comprising a guidance for evaluating body odor of a subject based on the olfactory sensitivity of the subject to the odor substance.
[34] Use of furfuryl mercaptan or coffee flavor for the evaluation of oral odor.

### Examples

Hereinafter, the present invention will be more specifically described in the following Examples. The odor substances used in the following Examples are shown in Table 1.

**[Table 1]**

| Odor substance | Cas No. | Source |
|---|---|---|
| Ethyl vanillin | 121-32-4 | Sigma-Aldrich |
| Guaiacol | 90-05-1 | Tokyo Chemical Industry Co., Ltd. |
| 3-Methyl-3-sulfanylhexan-1-ol (3M3SH) | 307964-23-4 | Apollo Scientific |
| Muscone | 541-91-3 | Tokyo Chemical Industry Co., Ltd. |
| Indole | 120-72-9 | Tokyo Chemical Industry Co., Ltd. |
| Furfuryl mercaptan | 98-02-2 | Sigma-Aldrich |

### Reference Example 1: Production of human olfactory receptor-expressing cells

### 1) Cloning of human olfactory receptor genes

Genes encoding the human olfactory receptors OR10G4, OR4S2, OR5P3, OR2W1, and OR1A1 (which are as set forth in SEQ ID NOs: 1, 2, 3, 4, and 5, respectively) were cloned. Each gene was cloned by PCR using human genomic DNA female (G1521: Promega) as a template, based on the sequence information registered in GenBank. Each gene amplified by PCR was incorporated into a pENTR vector (Invitrogen) in accordance with the manual, and by utilizing the NotI/AscI site present on the pENTR vector, it was recombined to the NotI/AscI site produced downstream of a Flag-Rho tag sequence on a pME18S vector.

### 2) Production of pME18S-human RTP1S vector

An RTP1S gene encoding RTP1S was incorporated into the EcoRI/XhoI site of a pME18S vector.

### 3) Production of olfactory receptor-expressing cells

HEK293 cells in which any one of the above-described human olfactory receptors was allowed to express, were produced. A reaction solution having the composition shown in Table 2 was prepared, and was then left to stand in a clean bench for 15 minutes. The resultant was added into each well of a 96-well plate (BD). Subsequently, the HEK293 cells (3 × 10⁵ cells/cm²) were seeded in an amount of 90 µL into each well, and were then cultured for 24 hours in an incubator in which 37°C and 5% CO₂ were retained. As a control, cells expressing no olfactory receptors (Mock) into which a pME18S vector containing no human olfactory receptor genes was incorporated were prepared in the same manner as that described above, and were then cultured in the same manner as that described above.

**[Table 2]**

| | |
|---|---|
| DMEM (Nacalai) | 10 µL |
| Human olfactory receptor gene (which is incorporated into pME18S vector with Flag-Rho tag added to N-terminus thereof) | 0.075 µg |
| pGL4.29 (fluc2P-CRE-hygro, Promega) | 0.03 µg |
| pGL4.75 (hRluc-CMV, Promega) | 0.03 µg |
| pME18S-human RTP1S vector | 0.03 µg |
| Lipofectamine 2000 (Invitrogen) or PEI MAX (Polyscience) | 0.4 µL |

### Reference Example 2: Luciferase assay

An olfactory receptor expressed in HEK293 cells conjugated with Gαs existing in the cells to activate adenylate cyclase, so as to increase the amount of cAMP in the cells. For the receptor response measurement in the present study, a luciferase reporter gene assay was applied, and an increase in the intracellular amount of cAMP was monitored as a luminescence value derived from a firefly luciferase gene (fluc2P-CRE-hygro). Further, a Renilla luciferase gene which had been fused downstream of a CMV promoter (hRluc-CMV) was simultaneously transfected, and was used as an internal standard for correcting errors in gene transfer efficiency or the number of cells.

The medium was removed from the culture produced in the above 3), and an odor substance solution was then added thereto. The odor substance solution was prepared using DMEM (Nacalai Tesque, Inc.), or DMEM (Nacalai Tesque, Inc.) containing 300 µM copper chloride. The cells were cultured in a CO₂ incubator for 4 hours, and the luciferase gene was allowed to sufficiently express in the cells. The activity of luciferase was measured using Dual-Glo^{™} luciferase assay system (Promega) in accordance with the operating manual of the product. The value fLuc/hRluc was calculated by dividing the value of luminescence derived from firefly luciferase by the value of luminescence derived from Renilla luciferase. A fold increase was calculated by dividing the value fLuc/hRluc induced by cells stimulated with an odor substance, by the value fLuc/hRluc induced by cells not stimulated with an odor substance, and the obtained value was used as an indicator of response intensity.

### Reference Example 3: Response of OR10G4 to odor substances

The response of OR10G4 to odor substances was measured according to the methods described in Reference Examples 1 and 2. As a result, it was confirmed that both ethyl vanillin and guaiacol activate OR10G4 (Figure 1). These results are consistent with the report described in Patent Literature 1.

### Example 1: Fluctuation in olfactory sensitivity by odor in oral cavity

Patent Literature 1 reports that when a subject had previously smelled ethyl vanillin, not only the olfactory sensitivity of the subject to ethyl vanillin, which the subject smelled later, weaken, but also the olfactory sensitivity of the subject to guaiacol also weakened due to cross-adaptation. In the present example, whether oral odor, namely, odor in the oral cavity causes adaptation and cross-adaptation to the sense of smell was verified. Specifically, whether the olfactory sensitivity of a subject to ethyl vanillin or guaiacol is reduced by addition of ethyl vanillin into the oral cavity was examined.

### 1) Method

A 0.5% (w/w) ethyl vanillin-containing tablet (0.5% EV tablet) was placed into the mouth of a subject, and the subject continuously licked the tablet. In this situation, a fluctuation in the olfactory sensitivity was examined. As odor substances used to evaluate olfactory sensitivity, ethyl vanillin and guaiacol known to have cross-adaptation with ethyl vanillin were used.

### (Preparation of test samples)

2.0 mg of ethyl vanillin (≥ 98%, Sigma-Aldrich) was placed into a glass bottle (Maruemu No. 6). This was used as a test sample for evaluation of ethyl vanillin. In addition, guaiacol (> 98%, Tokyo Chemical Industry Co., Ltd.) was diluted with mineral oil to prepare a 10 ppmv guaiacol solution, and a cotton ball (Hakujuji Co., Ltd., No. 10) having a diameter of approximately 10 mm was impregnated with 20 µL of this solution, and was then placed in a glass bottle (Maruemu No. 6). This was used as a test sample for evaluation of guaiacol.

### (Sensory test)

The sensory test was carried out with three subjects. In the test, first, the subjects had any of the test samples, and were evaluated in terms of odor sensory intensity to the sample. Subsequently, the subjects licked five 0.5% EV tablets, and then, at time points of 1 minute, 3 minutes, and 5 minutes after the licking, the subjects had the same test sample again, and odor sensory intensity to the sample was evaluated. These procedures are defined as 1 set of the test. Such 1 set of the test was carried out for each of two types of test samples. As a control, the odor sensory intensity of the subjects to two types of test samples was evaluated in the same procedures as those described above, with the exception that ethyl vanillin-free tablets (0% EV tablets) were given to the subjects. That is to say, a total of 4 sets of the test were carried out on each subject. Between the sets, an interval of at least 15 minutes was established. For the evaluation of odor sensory intensity, Visual analogue scale (VAS) was used.

Regarding each test sample, a relative value of the odor sensory intensity (VAS value) at 1 minute, 3 minutes, and 5 minutes after ingestion of the tablets was obtained, based on the odor sensory intensity (VAS value) before ingestion of the tablets that was set at 100%. A difference in relative sensory intensity between the test conditions (0.5% EV tablets) and the control conditions (0% EV tablets) at individual time points (before ingestion, 1 minute, 3 minutes, and 5 minutes) was compared by a multiple comparison test according to the Sidak method following 2-way ANOVA, and a multiplicity-adjusted p value was calculated. The statistical analysis was carried out using GraphPad prism 6 (MDF).

### 2) Results

The results of the sensory test are shown in Figure 2. As a result of the licking of ethyl vanillin-containing tablets, a statistically significant reduction in the odor sensory intensity to ethyl vanillin was observed, in comparison to the conditions of ethyl vanillin-free tablets. Moreover, even to guaiacol that is a substance exhibiting cross-adaptation with ethyl vanillin, a reduction in the odor sensory intensity was observed by the licking of ethyl vanillin-containing tablets. These results suggested that not only odor received through nasal cavity from the outside, but also odor generated in the oral cavity of a subject himself or herself, can reach the olfactory epithelium and can act on the sense of smell, and thus, can cause adaptation and cross-adaptation, thereby reducing the olfactory sensitivity.

### Example 2: Fluctuation in olfactory sensitivity by underarm odor

In the present example, whether underarm odor causes adaptation to the sense of smell was verified. Specifically, a representative underarm odor-causing substance, 3-methyl-3-sulfanylhexan-1-ol (3M3SH), was applied to the armpit portion of clothes and was then removed. At that time, a fluctuation in the olfactory sensitivity to 3M3SH over time was examined.

### 1) Method

### (Preparation of samples)

3M3SH was used as an underarm odor-causing substance. A sensory evaluation sample was prepared by adding one cotton ball (Hakujuji Co., Ltd., No. 10) into a 13.5-mL vial bottle (Maruemu No. 5), and then adding 10 µL of a 3M3SH solution with each diluted concentration thereto. For the 3M3SH solution, mineral oil (Sigma-Aldrich) was used as a solvent, and the concentrations of the solution were set to be 0 µM, 1 µM, 3 µM, 10 µM, 30 µM, 100 µM, and 300 µM.

### (Sensory test)

The sensory test was carried out with two subjects. The subjects wore a commercially available white cloth (material: 65% polyester and 35% cotton). The subjects first smelled the above-described samples in the order from a low concentration sample to a high concentration sample, and odor sensory intensity which the subjects felt was then evaluated. The evaluation was carried out in 9 stages from "0: Not felt" to "4: Unbearably strongly felt," at intervals of 0.5. Thereafter, the 3M3SH ethanol solution (3 µg/mL) was added in an amount of 10 µL each onto both of the armpit portions of each subject' clothes (white clothes). Fifteen minutes after addition of this solution, the above-described sensory evaluation was carried out again. Immediately after the second sensory evaluation, the 3M3SH ethanol solution was added again onto both of the armpit portions of the clothes in the same manner as that described above, and thereafter, addition of the 3M3SH solution onto both of the armpit portions of the clothes was carried out every 15 minutes, and the sensory evaluation was carried out every 30 minutes. When the timing of addition of the 3M3SH solution was overlapped with the timing of sensory evaluation, immediately after the sensory evaluation, the 3M3SH solution was added onto both of the armpit portions of the clothes. Ninety minutes after initiation of the test, the subjects took the white clothes off, and addition of the 3M3SH solution was terminated. Thereafter, the sensory evaluation was carried out until 165 minutes after initiation of the test, and the test was finally terminated. Besides, the amount of 3M3SH added to the clothes was set to be 240 ng per hour. According to the previous study (J Invest Dermatol. 2010, 130(2): 529-40), the maximum value of the 3M3SH precursor captured from both armpits for 30 minutes is described to be 0.051 µmol. In another previous study (FEMS Microbiol Lett. 2015, 362(16): fnv111), the maximum value of metabolism efficiency from the 3M3SH precursor to 3M3SH for 24 hours is described to be 44.6%, even though this is an *in vitro* result. Calculating from the above-described information, it could be estimated that, at maximum, approximately 281 ng of 3M3SH would be generated from both armpits per hour. Accordingly, in the present example, experimental conditions, which were not apart from real living scene, were provided by applying 240 ng of 3M3SH onto the armpit portions per hour.

### 2) Results

A change over time in the odor sensory intensity of 3M3SH is shown in Figure 3. Fifteen minutes after application of 3M3SH onto the armpit portions, the odor intensity of 3M3SH felt from the sample was reduced. Furthermore, the reduced sense of smell to the 3M3SH sample was recovered at least 15 minutes to 45 minutes after the removal of 3M3SH from the armpit portions. As described above, it was suggested that olfactory sensitivity to a specific odor substance should be regulated, not only by oral odor, but also by body odor including underarm odor, and that the olfactory sensitivity to the specific odor substance should be improved by removing body odor including underarm odor as a typical example.

### Example 3: olfactory receptors which recognizes oral odor substances and ligand selectivity thereof

As described in Example 1, an odor substance in the oral cavity acts on the olfactory epithelium to reduce olfactory sensitivity. On the other hand, in the oral cavity, a variety of oral odor substances are present. Table 3 shows the oral odor substances reported in the previous study (J Chromatogr B Analyt Technol Biomed Life Sci, 2007, 853(1-2): 54-61), and human olfactory receptors which respond to those oral odor substances, which were found in the previous study (US9914760B2, J Neurosci, 2009, 29(1): 153-158, Sci Data, 2015, 2: 150002, PLoS Biol, 2007, 30, 5(11): e284, PLoS One, 2013, 8(2): e54950) and the present study (Figure 4).

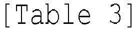

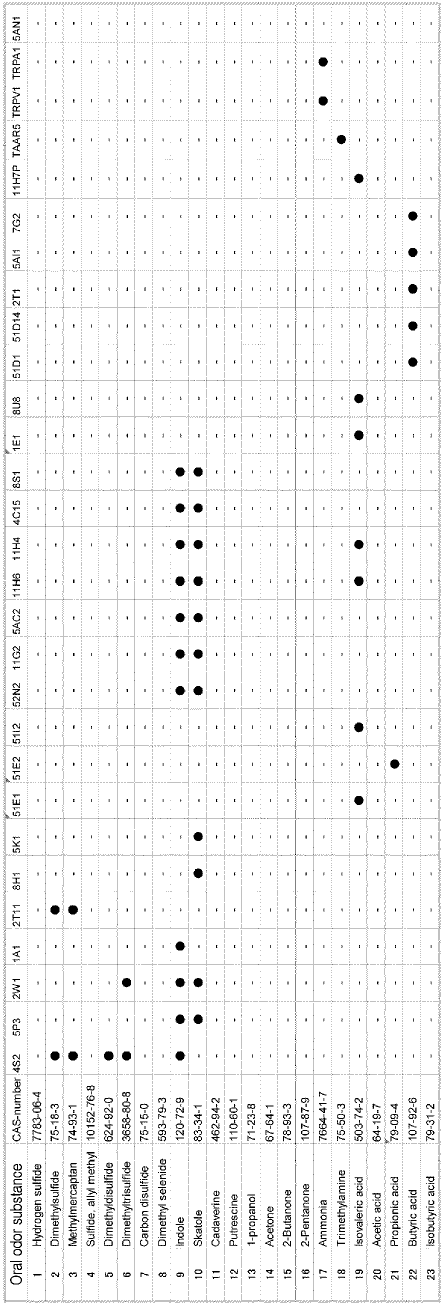

### Example 4: Recovery of olfactory sensitivity by oral care 1) Response of receptor to oral odor substance

In the present example, after a reduction in an oral odor substance by oral care, whether olfactory sensitivity to the oral odor substance and odor substances other than the oral odor substance is recovered was examined. Indole was used as a representative oral odor substance (J Chromatogr B Analyt Technol Biomed Life Sci, 2007, 853(1-2): 54-61), and furfuryl mercaptan and muscone were used as odor substances other than the oral odor substance. The response of olfactory receptors to these odor substances was measured according to the methods described in Reference Examples 1 and 2. As a result, it was revealed that the indole receptor OR4S2 is activated by furfuryl mercaptan, but is not activated by muscone (Figure 5).

Muscone is recognized, mainly mediated by OR5AN1 (Patent Literature 1). On the other hand, an oral odor substance which activates OR5AN1 has not yet been discovered at present (Table 3). Accordingly, it was predicted that, when oral odor substances including indole are reduced in the oral cavity after oral care, the responsiveness of OR5AN1 does not change, while the responsiveness of OR4S2 is recovered, and thereby, olfactory sensitivity to muscone does not change, and olfactory sensitivity to indole and furfuryl mercaptan increases.

### 2) Evaluation of olfactory sensitivity

Olfactory sensitivity before and after oral care was evaluated according to a sensory test. The experimental scheme is shown in Figure 6A, and is also described in detail below. The sensory test was carried out with 14 subjects. On the test day, the subjects were prohibited to eat and drink anything after getting up, and were also prohibited to do oral care including brushing of teeth. The test was started between 8:00 am to 9:30 am. First, exhaled breath was collected from the subjects. Subsequently, the detection threshold concentration of each odor substance was measured according to the sensory test. Thereafter, the subjects ate designated breakfast, and then performed oral care (the brushing of teeth using commercially available toothbrush and dentifrice for 5 minutes, and then, the washing of the mouth using commercially available mouth wash for 30 seconds). At individual time points of 10 minutes and 1 hour after the oral care, exhaled breath was collected from the subjects and the detection threshold concentration of each odor substance was measured.

### (Measurement of substance in exhaled breath)

The subjects entered their exhaled breath into an air sampling bag (GL Sciences Inc., PA AA-2). The exhaled breath sample was preserved at room temperature, and on the same day, it was subjected to analysis. Solid Phase Micro Extraction (SPME) Fiber (SPELCO, PDMS/DVB) was exposed into the sampling bag, and exhaled breath substances were captured for 1 hour. The indole concentration in the exhaled breath was calculated according to SPME-GC/MS. The indole concentrations before and after the oral care were compared with each other according to the Wilcoxon signed-rank test, and the p value was calculated. The statistical analysis was carried out using R Software (Ver.3.6.0). <GC/MS conditions>
GC: 7890A (Agilent Technologies Japan, Ltd.)
Injection condition: Splitless
Column: VF-WAXms
Carrier gas: Helium
Oven temperature: 40°C (4 min hold) → temperature increase at 6°C/min. → 240°C (2 min hold)
MS: 5975C (Agilent Technologies Japan, Ltd.)

### (Odor sample)

1 mL of an odor substance solution placed in a vial bottle (Maruemu, No. 2) was used as an odor sample. As such odor substance solutions, a 20-step 2-fold dilution series was prepared regarding each of muscone, indole, and furfuryl mercaptan (highest concentrations: 1000 ppm, 1 mM, and 10 ppb, respectively). Mineral oil (Sigma-Aldrich) was used as a solvent in all cases. As a blank, a vial containing 1 mL of mineral oil was used.

### (Measurement of detection threshold concentration)

A single test was carried out on two types of odor substance among muscone, indole, and furfuryl mercaptan. The subjects had a total of three vials (i.e. 2 blank vials and 1 vial containing an odor sample). The test participants smelled the odors contained in the vials and tried to choose the vial containing an odor solution. The test was started with an odor sample having the third low concentration. When the subject's selection was incorrect or the subject could not choose the vial even once, the same trial was carried out using an odor sample having one step higher concentration. When the subject' selection was correct two times in a row, the same trial was carried out using an odor sample having one step lower concentration. When the subject' selection was correct two times in a row using an odor sample with a new concentration, the subject was tested using an odor sample having further one step lower concentration. When the subject could not choose the correct vial even once, the subject was tested using an odor sample having one step higher concentration. Switching from a high concentration to a low concentration, and from a low concentration to a high concentration, was recorded 7 times in total, and the mean value of the concentrations measured 4 times in the second half was defined as a detection threshold concentration. The detection threshold concentrations of each odor substance before and after oral care were compared with each other according to the Wilcoxon signed-rank test, and the p value was calculated. The statistical analysis was carried out using R Software (Ver.3.6.0).

### 3) Results

### (Concentrations of substances in exhaled breath)

The indole concentration in the breath is shown in Figure 6B. Indole was detected from all of the subjects. The indole concentration was significantly reduced at 1 hour after the oral care, in comparison to before the oral care. These results show that the oral odor substance in the exhaled breath was removed by the oral care. Besides, muscone and furfuryl mercaptan were not detected both before and after the oral care, and these results were consistent with no detection of muscone and furfuryl mercaptan according to the reports from a large number of previous studies regarding the analysis of oral odor substances.

### (Fluctuation in olfactory sensitivity)

The results of the measurement of detection threshold concentrations before and after the oral care according to the sensory evaluation are shown in Figure 7. There was no statistically significant difference between before oral care and 1 hour after the oral care, in terms of olfactory sensitivity to muscone (Figure 7A, left). It was considered that these results were provided because both muscone and a substance which activates the muscone receptor OR5AN1 are not included in the oral odor substances for the first place, and thus, even though the oral odor substances are reduced by the oral care, the responsiveness of OR5AN1 does not change. In contrast, the olfactory sensitivity to indole as an oral odor substance was statistically significantly improved 1 hour after the oral care, in comparison to before the oral care (Figure 7A, center). An increase in sensitivity to indole was observed in all of the subjects. Besides, in comparison to before the oral care, the detection threshold concentration of indole was decreased to 1/8 times on average 1 hour after the oral care (Figure 7A, center), and was decreased to 1/16 times 10 minutes after the oral care (Figure 7B). In addition, although furfuryl mercaptan which activates the indole olfactory receptor OR4S2 is not an oral odor substance, the sensitivity of the subjects to furfuryl mercaptan was statistically significantly improved 1 hour after the oral care, in comparison to before the oral care, and this sensitivity improvement was observed in all of the subjects (Figure 7A, right). Therefore, it was considered that the responsiveness of OR4S2 was recovered together with a reduction in indole in the oral cavity, and that the olfactory sensitivity of the subjects to both indole and furfuryl mercaptan which activate OR4S2 was improved.

Since oral odor is constantly present in the oral cavity, it has been assumed so far that the individual's sense of smell would have long-term adaptation to the oral odor. Since recovery from long-term adaptation requires a long period of time that is equivalent to the adaptation period (Non Patent Literatures 2 to 4), it was predicted that the recovery of olfactory sensitivity to oral odor after the removal of the oral odor would also require a long period of time. In contrast, the results of the present example showed for the first time that the subjects recover, at a clear level, from adaptation to the most familiar odor that is oral odor, in a non-exposure time of only 10 minutes to 1 hour. This is a finding, which contradicts the above-described previous prediction that is based on the understanding that a long non-exposure time is required for the recovery from adaptation due to long-term exposure to odor. Moreover, from the results of the present example, it was revealed that the type of an odor substance to which olfactory sensitivity increases in a short time after the oral care could be predicted from the viewpoint that is "a substance recognized by an olfactory receptor which responds to the oral odor substance."

### Example 5: Improvement of sensitivity to coffee flavor due to oral care

Furfuryl mercaptan is a main flavor component of coffee. Whether the coffee flavor can be more sufficiently felt, when oral odor is reduced by oral cavity, was verified.

### 1) Method

The sensory test was carried out with three subjects. The test was carried out for two consecutive days, and the test was started from 8:40 am on both days. On the test day, the subjects were prohibited to eat and drink anything after getting up, and were also prohibited to do oral care including brushing of teeth. On the first day, approximately 150 mL of commercially available instant coffee adjusted to approximately 80°C was given to the subjects, so that they were allowed to perform sensory evaluation. On the second day, before performing sensory evaluation of coffee, the subjects performed oral care for 2 minutes, using commercially available toothbrush and dentifrice, and then took a rest for 5 minutes. Thereafter, in the same manner as that on the first day, approximately 150 mL of commercially available instant coffee adjusted to approximately 80°C was given to the subjects, so that they were allowed to perform sensory evaluation. The score on the first day was compared with the score on the second day.

### (Sensory evaluation)

Coffee was prepared immediately before the sensory evaluation, so that the flavor of the coffee was not impaired. Before the sensory evaluation using coffee, the subjects first drank a sip of water, and thereafter, they drank the coffee and answered the questionnaire of the sensory evaluation. In the questionnaire of the sensory evaluation, the subjects evaluated the "intensity of aroma," "intensity of bitterness," "intensity of sourness," "intensity of sweetness," "intensity of richness," "intensity of aftertaste," and "intensity of coffee flavor" of the coffee, according to the five-point scale (1: not felt; 2: not felt so much; 3: felt; 4: strongly felt; and 5: extremely strongly felt). The mean value of the evaluation scores of the three subjects was obtained.

### 2) Results

The results of the sensory evaluation are shown in Figure 8. It was found that coffee flavor tended to be felt more strongly in the case with oral care, than in the case without oral care. Therefore, it was demonstrated that the improvement of olfactory sensitivity by a reduction in oral odor is so significant that it clearly has effects on the impression of flavor in the daily life.

### Example 6: Oral odor substances and olfactory receptor group which responds thereto

As shown in Table 3, there are a variety of oral odor substances are present in the oral cavity, and a large number of olfactory receptors which respond to those oral odor substances are also present. For example, "#22 Butyric acid" shown in Table 3 is considered to be an oral odor substance that increases with hepatic dysfunction, and this mouse odor substance is recognized by OR51D1, OR51D14, OR2T1, OR5Al1, and OR7G2. Therefore, it is considered that a reduction in such an oral odor substance in the oral cavity will provide the results that the sensitivity of these olfactory receptors is improved in a short time.

Further as described in Examples 3 and 4, the improvement of the responsiveness of an oral odor receptor group leads to the improvement of the sensitivity to various odor substances other than oral odor substances. For example, Figure 9 shows the ligand spectra of the four indole receptors OR4S2, OR5P3, OR2W1 and OR1A1, which were examined according to the methods described in Reference Examples 1 and 2. Since OR4S2 is activated by odor substances classified into sulfides and thiols based on their chemical structure, it is considered that the improvement of the responsiveness of OR4S2 by oral care will lead to clear sensitivity to the odor substances classified into sulfides and thiols. Moreover, since OR2W1 may be associated with the recognition of a wide range of odor substances other than sulfides, it is considered that the improvement of the sensitivity of OR2W1 by oral care will be involved in the sensitivity to such a wide range of odor substances. Likewise, even in the case of body odor other than oral odor, by improving the responsiveness of olfactory receptors which respond to the body odor by body odor care, it is considered that the olfactory sensitivity to a wide range of odor substances to which these olfactory receptors respond will be improved.

## Claims

1. A method for immediately improving the olfactory sensitivity of a subject, comprising reducing body odor of the subject.

2. The method according to claim 1, comprising removing a body odor-causing substance from body or clothes of the subject.

3. The method according to claim 1 or 2, wherein the body odor is oral odor.

4. The method according to claim 3, comprising washing oral cavity.

5. The method according to claim 3 or 4, wherein the olfactory sensitivity is sensitivity to coffee flavor.

6. The method according to claim 1 or 2, wherein the body odor is underarm odor.

7. The method according to claim 6, comprising washing the armpit portion.

8. A method for selecting an agent for immediately improving olfactory sensitivity, comprising specifying a substance which removes a body odor-causing substance.

9. The method according to claim 8, further comprising performing sensory evaluation of the olfactory sensitivity-improving action of the specified substance which removes a body odor-causing substance.

10. A method for evaluating body odor, comprising measuring olfactory sensitivity of a subject.

11. The method according to claim 10, wherein the body odor is oral odor.

12. The method according to claim 11, wherein the olfactory sensitivity is sensitivity to furfuryl mercaptan or coffee flavor.

13. The method according to claim 10, wherein the body odor is underarm odor.

14. A kit for evaluating body odor, comprising a means for measuring olfactory sensitivity of a subject.

15. The kit according to claim 14, wherein the body odor is oral odor.

16. The kit according to claim 15, wherein the olfactory sensitivity is sensitivity to furfuryl mercaptan or coffee flavor.

17. The kit according to claim 14, wherein the body odor is oral odor.

18. Use of a substance which removes an oral odor-causing substance from the oral cavity, for immediately improving olfactory sensitivity.

19. The use according to claim 18, wherein the olfactory sensitivity is sensitivity to coffee flavor.

20. Use of furfuryl mercaptan or coffee flavor for evaluating oral odor.

21. Use of a substance which removes an underarm odor-causing substance from the body, for immediately improving olfactory sensitivity.
